# EUROPEAN PATENT SPECIFICATION

(11) **EP 3 908 194 B1**
(45) Date of publication and mention of the grant of the patent: **18.02.2026**
(21) Application number: 19829227.8
(22) Date of filing: 24.12.2019
(51) Int. Cl.: A61B 8/12, A61B 8/00

(54) **INCREASED FLEXIBILITY SUBSTRATE FOR INTRALUMINAL ULTRASOUND IMAGING ASSEMBLY**
SUBSTRAT MIT ERHÖHTER FLEXIBILITÄT FÜR INTRALUMINALE ULTRASCHALLBILDGEBUNGSVORRICHTUNG
SUBSTRAT À SOUPLESSE RENFORCÉE POUR ENSEMBLE D'IMAGERIE ULTRASONORE INTRALUMINAL

(30) Priority: 07.01.2019 US 201962789099 P
(43) Date of publication of application: 17.11.2021
(73) Proprietor: Koninklijke Philips N.V., 5656 AG Eindhoven (NL)
(72) Inventor: MINAS, Maritess, 5656 AE Eindhoven (NL); WROLSTAD, David, Kenneth, 5656 AE Eindhoven (NL)
(74) Representative: Philips Intellectual Property & Standards
(86) International application number: PCT/EP2019/086999
(87) International publication number: WO 2020/144070

(56) References cited:
- EP-A1- 3 274 040
- EP-A2- 0 853 919
- EP-B1- 0 853 919
- WO-A1-2019/002231
- GB-A- 2 315 020
- US-A1- 2016 279 388

## Description

### TECHNICAL FIELD

The present disclosure relates generally to intraluminal ultrasound imaging and, in particular, to the structure of an ultrasound imaging assembly at a distal portion of a catheter or guidewire. For example, a flexible substrate of an ultrasound imaging assembly includes recesses that increase its flexibility to allow for efficient transitioning into a rolled configuration from a flat configuration.

### BACKGROUND

Intravascular ultrasound (IVUS) imaging is widely used in interventional cardiology as a diagnostic tool for assessing a diseased vessel, such as an artery, within the human body to determine the need for treatment, to guide the intervention, and/or to assess a treatment's effectiveness. An IVUS device including one or more ultrasound transducers is passed into the vessel and guided to the area to be imaged. The transducers emit ultrasonic energy in order to create an image of the vessel of interest. Ultrasonic waves are partially reflected by discontinuities arising from tissue structures (such as the various layers of the vessel wall), red blood cells, and other features of interest. Echoes from the reflected waves are received by the transducer and passed along to an IVUS imaging system. The imaging system processes the received ultrasound echoes to produce a cross-sectional image of the vessel where the device is placed.

Solid-state (also known as synthetic-aperture) IVUS catheters are one of the two types of IVUS devices commonly used today, the other type being the rotational IVUS catheter. Solid-state IVUS catheters carry a scanner assembly that includes an array of ultrasound transducers distributed around its circumference along with one or more integrated circuit controller chips mounted adjacent to the transducer array. The controllers select individual acoustic elements (or groups of elements) for transmitting an ultrasound pulse and for receiving the ultrasound echo signal. By stepping through a sequence of transmit-receive pairs, the solid-state IVUS system can synthesize the effect of a mechanically scanned ultrasound transducer but without moving parts (hence the solid-state designation). Since there is no rotating mechanical element, the transducer array can be placed in direct contact with the blood and vessel tissue with minimal risk of vessel trauma. Furthermore, because there is no rotating element, the electrical interface is simplified. The solid-state scanner can be wired directly to the imaging system with a simple electrical cable and a standard detachable electrical connector, rather than the complex rotating electrical interface required for a rotational IVUS device.

Existing solid-state devices present several challenges. The electrical cable is attached to a flex circuit of an IVUS imaging assembly close to electronic components. Attaching the cables in such close proximity can potentially harm operation of the electronic components. The cable connection also increases the stiff length at the distal portion of a catheter, which reduces the catheter's ability to traverse tortuous vasculature. Ensuring that conductive traces formed in the flex circuit stay operational while being handled during the manufacturing process is also a challenge. Assembly of a solid-state IVUS device sometimes requires a flex circuit to be rolled around the circumference of the catheter. Such steps during the manufacturing can be difficult to automate in a reproducible manner because of the added thickness of some portions of the flex circuit.

US 2016/0279388 Al, according to its abstract, relates to articulation devices, systems, methods for articulation, and methods for fabricating articulation structures. These will often include simple balloon arrays, with inflation of the balloons interacting with elongate skeletal support structures so as to locally alter articulation of the skeleton. The balloons can be mounted to a substrate of the array, with the substrate having channels that can direct inflation fluid to a subset of the balloons. The articulation array structure may be formed using simple planar 3-D printing, extrusion, and/or laser micromachining techniques. The skeleton may comprise a simple helical coil or interlocking helical channels, and the array can be used to locally deflect or elongate an axis of the coil under control of a processor. Liquid inflation fluid may be directed to the balloons from an inflation fluid canister, and may vaporize within the channels or balloons of the articulation system, with the inflation system preferably including valves controlled by the processor. The articulation structures can be employed in minimally invasive medical catheter systems, and also for industrial robotics, for supporting imaging systems, for entertainment and consumer products, and the like.

### SUMMARY

The object of the present invention is solved by the subject-matter of the independent claims; further embodiments are incorporated in the dependent claims.

An intraluminal imaging device, such as an intravascular ultrasound (IVUS) imaging catheter, is described herein. The ultrasound imaging assembly at the distal portion of the catheter includes a flexible substrate. The flexible substrate has a distal portion with acoustic elements positioned thereon, as well as a proximal portion including weld pads to which electrical conductors are attached. The proximal portion is thicker than the distal portion because it includes an additional layer to protect conductive traces that allow electrical communication. In order to counteract the stiffness resulting from the increased thickness (e.g., not being able to bend to as tight a radius as the distal portion), recesses extending completely through the proximal portion of the flexible substrate are provided. These recesses remove material in a manner that does not interfere with operation of the device, and return flexibility to the proximal portion. The flexibility allows for the substrate to be efficiently transitioned from a flat configuration into a rolled configuration (e.g., a cylindrical for the distal portion and a spiral configuration from the proximal portion).

In an exemplary embodiment, an intraluminal imaging device is provided. The device includes a flexible elongate member configured to be positioned within a body lumen of a patient, the flexible elongate member comprising a proximal portion and a distal portion; an ultrasound imaging assembly disposed at the distal portion of the flexible elongate member, the ultrasound imaging assembly comprising: a flexible substrate comprising: a proximal portion comprising a plurality of recesses extending completely through the flexible substrate from a first surface to an opposite, second surface; and a distal portion comprising a plurality of acoustic elements; a support member around which the distal portion of the flexible substrate is positioned; and a plurality of conductors extending along a length of the flexible elongate member and coupled to the proximal portion of the flexible substrate such that the plurality of conductors are in communication with the plurality of acoustic elements.

In some embodiments, the proximal portion of the flexible substrate comprises a first thickness greater than a second thickness of the distal portion of the flexible substrate. In some embodiments, the distal portion of the flexible substrate comprises a first layer, and the proximal portion of the flexible substrate comprises the first layer and a second layer. In some embodiments, the first layer comprises the first surface and the second layer comprises the second surface such that the plurality of recesses extend completely through the first layer and the second layer. In some embodiments, the first layer and the second layer comprise a same material.

In some embodiments, the distal portion of the flexible substrate comprises a plurality of integrated circuit chips in communication with the plurality of acoustic elements, and a first plurality of conductive traces providing communication between the plurality of integrated circuit chips and the plurality of acoustic elements, wherein the proximal portion of the flexible substrate comprises a plurality of conductive pads at which the plurality of conductors are coupled, respectively, and a second plurality of conductive traces providing communication between the plurality of conductive pads and the plurality of integrated circuit chips. In some embodiments, the plurality of recesses are spaced apart from one another in the proximal portion of the flexible substrate between the second plurality of conductive traces. In some embodiments, the plurality of recesses are arranged in a same orientation as the second plurality of conductive traces. In some embodiments, the proximal portion of the flexible substrate comprises one or more electrical components, wherein each of the one or more electrical components is disposed along a path of a respective conductive trace of the second plurality of conductive traces. In some embodiments, the proximal portion of the flexible substrate comprises a first width less than a second width of the distal portion of the flexible substrate. In some embodiments, the distal portion of the flexible substrate comprises a cylindrical configuration around the support member, and wherein the proximal portion of the flexible substrate comprises a spiral configuration. In some embodiments, the flexible elongate member comprises an inner member, wherein the proximal portion of the flexible substrate comprises the spiral configuration around the inner member. In some embodiments, the spiral configuration is trained into the proximal portion of the flexible substrate by either or both of heat or compression. In some embodiments, the proximal portion of the flexible substrate extends at an oblique angle relative to the distal portion of the flexible substrate.

In an exemplary embodiment, a method of assembling an intraluminal imaging device is provided. The method includes: providing an ultrasound imaging assembly comprising a flexible substrate in a flat configuration, the flexible substrate comprising a distal portion comprising a plurality of acoustic elements, and a proximal portion comprising a plurality of recesses extending completely through the flexible substrate from a first surface of the flexible substrate to an opposite, second surface of the flexible substrate; transitioning the flexible substrate from the flat configuration into a rolled configuration, wherein the plurality of recesses increase a flexibility of the proximal portion for the proximal portion to transition into the rolled configuration; coupling the ultrasound imaging assembly to a distal portion of a flexible elongate member configured to be inserted into a body lumen of a patient; and establishing communication between the plurality of acoustic elements and a plurality of conductors extending along a length of the flexible elongate member, wherein establishing communication comprises coupling the plurality of electrical conductors to the proximal portion of the flexible substrate.

In some embodiments, transitioning comprises rolling the distal portion of the flexible substrate into a cylindrical configuration, and rolling the proximal portion of the flexible substrate into a spiral configuration. In some embodiments, transitioning comprises training the proximal portion of flexible substrate to retain the spiral configuration. In some embodiments, training comprises inserting the proximal portion of the flexible substrate into a heat shrink mold, and applying heat such that the heat shrink mold compresses the proximal portion of the flexible substrate in the spiral configuration.

Additional aspects, features, and advantages of the present disclosure will become apparent from the following detailed description.

### BRIEF DESCRIPTION OF THE DRAWINGS

Illustrative embodiments of the present disclosure will be described with reference to the accompanying drawings, of which:
Figure 1 is a diagrammatic schematic view of an intraluminal imaging system, according to aspects of the present disclosure.
Figure 2 is a diagrammatic view of the top of a scanner assembly in a flat configuration, according to aspects of the present disclosure.
Figure 3 is a diagrammatic perspective view of the scanner assembly shown in Figure 2 in a rolled configuration around a support member, according to aspects of the present disclosure.
Figure 4 is a diagrammatic cross-sectional side view of a scanner assembly in a rolled configuration around a support member, according to aspects of the present disclosure.
Figure 5 is a diagrammatic side view of an ultrasound imaging assembly with a distal portion of a flexible substrate in a rolled configuration around a support member, according to aspects of the present disclosure.
Figure 6 is a top view of an ultrasound imaging assembly a flat or unrolled configuration, according to aspects of the present disclosure.
Figure 7a is a diagrammatic side view of a flexible substrate of an ultrasound imaging assembly, according to aspects of the present disclosure.
Figure 7b is a diagrammatic side view of the flexible substrate of Figure 7a, including recesses at a proximal portion, according to aspects of the present disclosure .
Figure 8 is a diagrammatic view of an ultrasound imaging assembly with a distal portion of a flexible substrate in a rolled configuration around a support member and a proximal portion including recesses, according to aspects of the present disclosure.
Figure 9 is a diagrammatic view of the proximal portion of the flexible substrate of Figure 8.
Figure 10 is a diagrammatic perspective view of a distal portion of an intraluminal imaging device, including an ultrasound imaging assembly, according to aspects of the present disclosure.
Figure 11 is a diagrammatic perspective view of a distal portion of an intraluminal imaging device, including an ultrasound imaging assembly, according to aspects of the present disclosure.
Figure 12 is a flow diagram of a method of assembling an intraluminal imaging device, according to aspects of the present disclosure.
Figure 13 is a top view of an ultrasound imaging assembly and a support member, according to aspects of the present disclosure.
Figure 14 is a top view of a support member positioned on top of a flexible substrate of an ultrasound imaging assembly, according to aspects of the present disclosure.
Figure 15 is a top view of a distal portion of a flexible substrate of an ultrasound imaging assembly inserted into a heat shrink mold, according to aspects of the present disclosure.
Figure 16 is a diagrammatic side view of an ultrasound imaging assembly inserted into a heat shrink mold, according to aspects of the present disclosure.
Figure 17 is a diagrammatic side view of an ultrasound imaging assembly in which a heated die is closed around the proximal portion a heat shrink mold, according to aspects of the present disclosure.
Figure 18 is a diagrammatic side view of an ultrasound imaging assembly with a proximal portion of a flexible substrate trained in a spiral configuration, according to aspects of the present disclosure.
Figure 19 is a diagrammatic side view of an ultrasound imaging assembly with a plurality of conductors coupled to a proximal portion of a flexible substrate, according to aspects of the present disclosure.
Figure 20 is a diagrammatic side view of a distal portion of an intraluminal imaging device, according to aspects of the present disclosure.

### DETAILED DESCRIPTION

For the purposes of promoting an understanding of the principles of the present disclosure, reference will now be made to the embodiments illustrated in the drawings, and specific language will be used to describe the same. It is nevertheless understood that no limitation to the scope of the disclosure is intended. Any alterations and further modifications to the described devices, systems, and methods, and any further application of the principles of the present disclosure are fully contemplated and included within the present disclosure as would normally occur to one skilled in the art to which the disclosure relates. In particular, it is fully contemplated that the features, components, and/or steps described with respect to one embodiment may be combined with the features, components, and/or steps described with respect to other embodiments of the present disclosure. For the sake of brevity, however, the numerous iterations of these combinations will not be described separately.

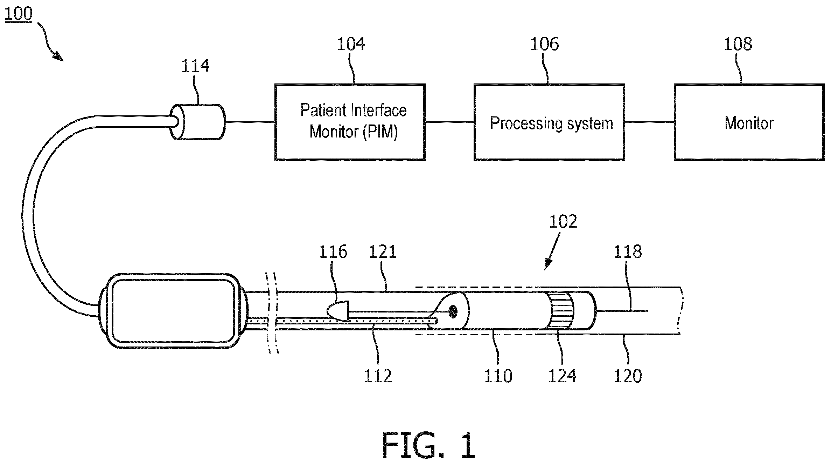

**Figure 1** is a diagrammatic schematic view of an ultrasound imaging system 100, according to aspects of the present disclosure. The ultrasound imaging system 100 can be an intraluminal imaging system. In some instances, the system 100 can be an intravascular ultrasound (IVUS) imaging system. The system 100 may include an intraluminal imaging device 102 such as a catheter, guide wire, or guide catheter, a patient interface module (PIM) 104, a processing system or console 106, and a monitor 108. The intraluminal imaging device 102 can be an ultrasound imaging device. In some instances, the device 102 can be an IVUS imaging device, such as a solid-state IVUS device.

At a high level, the IVUS device 102 emits ultrasonic energy, or ultrasound signals, from a transducer array 124 included in scanner assembly 110 mounted near a distal end of the catheter device. The ultrasonic energy is reflected by tissue structures in the medium, such as a vessel 120, or another body lumen surrounding the scanner assembly 110, and the ultrasound echo signals are received by the transducer array 124. In that regard, the device 102 can be sized, shaped, or otherwise configured to be positioned within the body lumen of a patient. The PIM 104 transfers the received echo signals to the console or computer 106 where the ultrasound image (including the flow information) is reconstructed and displayed on the monitor 108. The console or computer 106 can include a processor and a memory. The computer or computing device 106 can be operable to facilitate the features of the IVUS imaging system 100 described herein. For example, the processor can execute computer readable instructions stored on the non-transitory tangible computer readable medium.

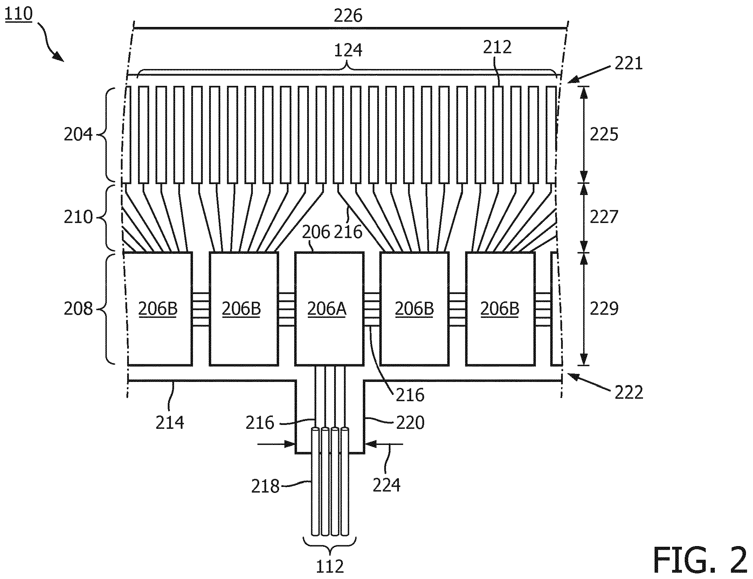

The PIM 104 facilitates communication of signals between the IVUS console 106 and the scanner assembly 110 included in the IVUS device 102. This communication includes the steps of: (1) providing commands to integrated circuit controller chip(s) 206A, 206B, illustrated in Figure 2, included in the scanner assembly 110 to select the particular transducer array element(s), or acoustic element(s), to be used for transmit and receive, (2) providing the transmit trigger signals to the integrated circuit controller chip(s) 206A, 206B included in the scanner assembly 110 to activate the transmitter circuitry to generate an electrical pulse to excite the selected transducer array element(s), and/or (3) accepting amplified echo signals received from the selected transducer array element(s) via amplifiers included on the integrated circuit controller chip(s) 206 of the scanner assembly 110. In some embodiments, the PIM 104 performs preliminary processing of the echo data prior to relaying the data to the console 106. In examples of such embodiments, the PIM 104 performs amplification, filtering, and/or aggregating of the data. In an embodiment, the PIM 104 also supplies high- and low-voltage DC power to support operation of the device 102 including circuitry within the scanner assembly 110.

The IVUS console 106 receives the echo data from the scanner assembly 110 by way of the PIM 104 and processes the data to reconstruct an image of the tissue structures in the medium surrounding the scanner assembly 110. Generally, the device 102 can be utilized within any suitable anatomy and/or body lumen of the patient. The processing system 106 outputs image data such that an image of the vessel or lumen 120, such as a cross-sectional IVUS image of the lumen 120, is displayed on the monitor 108. Lumen 120 may represent fluid filled or surrounded structures, both natural and man-made. Lumen 120 may be within a body of a patient. Lumen 120 may be a blood vessel, as an artery or a vein of a patient's vascular system, including cardiac vasculature, peripheral vasculature, neural vasculature, renal vasculature, and/or or any other suitable lumen inside the body. For example, the device 102 may be used to examine any number of anatomical locations and tissue types, including without limitation, organs including the liver, heart, kidneys, gall bladder, pancreas, lungs; ducts; intestines; nervous system structures including the brain, dural sac, spinal cord and peripheral nerves; the urinary tract; as well as valves within the blood, chambers or other parts of the heart, and/or other systems of the body. In addition to natural structures, the device 102 may be used to examine man-made structures such as, but without limitation, heart valves, stents, shunts, filters and other devices.

In some embodiments, the IVUS device includes some features similar to traditional solid-state IVUS catheters, such as the EagleEye^{®} catheter available from Volcano Corporation and those disclosed in U.S. Patent No. 7,846,101. For example, the IVUS device 102 includes the scanner assembly 110 near a distal end of the device 102 and a transmission line bundle 112 extending along the longitudinal body of the device 102 within a flexible elongate member 121. It is understood that any suitable gauge wire can be used for the conductors 218. In an embodiment, the cable 112 can include a four-conductor transmission line arrangement with, e.g., 41 AWG gauge wires. In an embodiment, the cable 112 can include a seven-conductor transmission line arrangement utilizing, e.g., 44 AWG gauge wires. In some embodiments, 43 AWG gauge wires can be used.

The transmission line bundle 112 terminates in a PIM connector 114 at a proximal end of the device 102. The PIM connector 114 electrically couples the transmission line bundle 112 to the PIM 104 and physically couples the IVUS device 102 to the PIM 104. In an embodiment, the IVUS device 102 further includes a guide wire exit port 116. Accordingly, in some instances the IVUS device is a rapid-exchange catheter. The guide wire exit port 116 allows a guide wire 118 to be inserted towards the distal end in order to direct the device 102 through the vessel 120.

In an embodiment, the image processing system 106 generates flow data by processing the echo signals from the IVUS device 102 into Doppler power or velocity information. The image processing system 106 may also generate B-mode data by applying envelope detection and logarithmic compression on the conditioned echo signals. The processing system 106 can further generate images in various views, such as 2D and/or 3D views, based on the flow data or the B-mode data. The processing system 106 can also perform various analyses and/or assessments. For example, the processing system 106 can apply virtual histology (VH) techniques, for example, to analyze or assess plaques within a vessel (e.g., the vessel 120). The images can be generated to display a reconstructed color-coded tissue map of plaque composition superimposed on a cross-sectional view of the vessel.

In an embodiment, the processing system 106 can apply a blood flow detection algorithm (e.g., ChromaFlo) to determine the movement of blood flow, for example, by acquiring image data of a target region (e.g., the vessel 120) repeatedly and determining the movement of the blood flow from the image data. The blood flow detection algorithm operates based on the principle that signals measured from vascular tissue are relatively static from acquisition to acquisition, whereas signals measured from blood flow vary at a characteristic rate corresponding to the flow rate. As such, the blood flow detection algorithm may determine movements of blood flow based on variations in signals measured from the target region between repeated acquisitions. To acquire the image data repeatedly, the processing system 106 may control to the device 102 to transmit repeated pulses on the same aperture.

An ultrasound transducer array of ultrasound imaging device includes an array of acoustic elements configured to emit ultrasound energy and receive echoes corresponding to the emitted ultrasound energy. In some instances, the array may include any number of ultrasound transducer elements. For example, the array can include between 2 acoustic elements and 10000 acoustic elements, including values such as 2 acoustic elements, 4 acoustic elements, acoustic elements, 64 acoustic elements, 128 acoustic elements, 500 acoustic elements, 812 acoustic elements, 3000 acoustic elements, 9000 acoustic elements, and/or other values both larger and smaller. In some instances, the transducer elements of the array may be arranged in any suitable configuration, such as a linear array, a planar array, a curved array, a curvilinear array, a circumferential array, an annular array, a phased array, a matrix array, a one-dimensional (1D) array, a 1.x dimensional array (e.g., a 1.5D array), or a two-dimensional (2D) array. The array of transducer elements (e.g., one or more rows, one or more columns, and/or one or more orientations) can be uniformly or independently controlled and activated. The array can be configured to obtain one-dimensional, two-dimensional, and/or three-dimensional images of patient anatomy.

The ultrasound transducer elements may comprise piezoelectric/piezoresistive elements, piezoelectric micromachined ultrasound transducer (PMUT) elements, capacitive micromachined ultrasound transducer (CMUT) elements, and/or any other suitable type of ultrasound transducer elements. The ultrasound transducer elements of the array are in communication with (e.g., electrically coupled to) electronic circuitry. For example, the electronic circuitry can include one or more transducer control logic dies. The electronic circuitry can include one or more integrated circuits (IC), such as application specific integrated circuits (ASICs). In some embodiments, one or more of the ICs can comprise a microbeamformer (µBF). In other embodiments, one or more of the ICs comprises a multiplexer circuit (MUX).

**Figure 2** is a diagrammatic top view of a portion of a scanner assembly 110 formed on a flexible substrate 214, according to aspects of the present disclosure. The scanner assembly 110 includes a transducer array 124 formed in a transducer region 204 and transducer control logic dies 206 (including dies 206A and 206B) formed in a control region 208, with a transition region 210 disposed therebetween.

The transducer control logic dies 206 are mounted on a flexible substrate 214 into which the transducers 212 have been previously integrated. The flexible substrate 214 is shown in a flat configuration in Figure 2. Though six control logic dies 206 are shown in Figure 2, any number of control logic dies 206 may be used. For example, one, two, three, four, five, six, seven, eight, nine, ten, or more control logic dies 206 may be used.

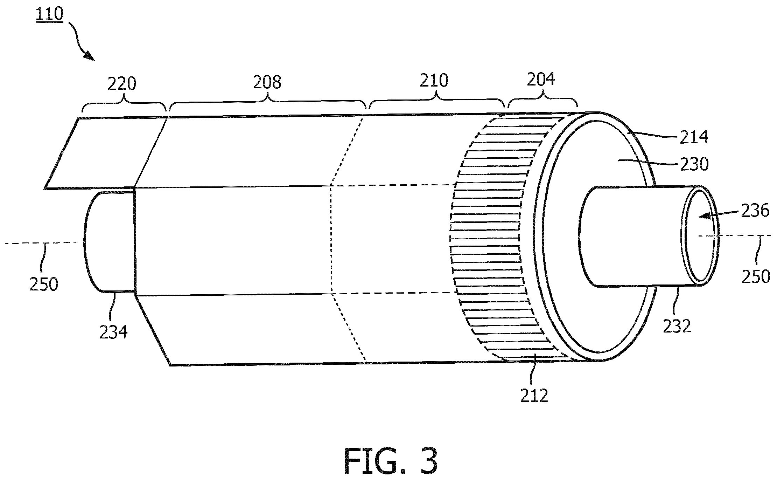

The flexible substrate 214, on which the transducer control logic dies 206 and the transducers 212 are mounted, provides structural support and interconnects for electrical coupling. The flexible substrate 214 may be constructed to include a film layer of a flexible polyimide material such as KAPTON^{™} (trademark of DuPont). Other suitable materials include polyester films, polyimide films, polyethylene napthalate films, or polyetherimide films, liquid crystal polymer, other flexible printed semiconductor substrates as well as products such as Upilex^{®} (registered trademark of Ube Industries) and TEFLON^{®} (registered trademark of E.I. du Pont). In the flat configuration illustrated in Figure 2, the flexible substrate 214 has a generally rectangular shape. As shown and described herein, the flexible substrate 214 is configured to be wrapped around a support member 230 (Figure 3) in some instances. Therefore, the thickness and flexibility of the film layer of the flexible substrate 214 is generally related to the degree of curvature in the final assembled flexible assembly 110. In some embodiments, the film layer is between 5 µm and 100 µm, with some particular embodiments being between 5 µm and 25.1 µm, e.g., 6 µm.

The transducer control logic dies 206 is a non-limiting example of a control circuit. The transducer region 204 is disposed at a distal portion 221 of the flexible substrate 214. The control region 208 is disposed at a proximal portion 222 of the flexible substrate 214. The transition region 210 is disposed between the control region 208 and the transducer region 204. Dimensions of the transducer region 204, the control region 208, and the transition region 210 (e.g., lengths 225, 227, 229) can vary in different embodiments. In some embodiments, the lengths 225, 227, 229 can be substantially similar or, the length 227 of the transition region 210 may be less than lengths 225 and 229, the length 227 of the transition region 210 can be greater than lengths 225, 229 of the transducer region and controller region, respectively.

The control logic dies 206 are not necessarily homogenous. In some embodiments, a single controller is designated a master control logic die 206A and contains the communication interface for transmission line bundle or cable 112 which may serve as electrical conductor(s), e.g., electrical conductor(s) 218, between a processing system, e.g., processing system 106, and the flexible scanner assembly 110. Accordingly, the master control circuit may include control logic that decodes control signals received over the cable or transmission line bundle 112, transmits control responses over the cable 142, amplifies echo signals, and/or transmits the echo signals over the cable or transmission line bundle 112. The remaining controllers are slave controllers 206B. The slave controllers 206B may include control logic that drives a transducer 212 to emit an ultrasonic signal and selects a transducer 212 to receive an echo. In the depicted embodiment, the master controller 206A does not directly control any transducers 212. In other embodiments, the master controller 206A drives the same number of transducers 212 as the slave controllers 206B or drives a reduced set of transducers 212 as compared to the slave controllers 206B. In an exemplary embodiment, a single master controller 206A and eight slave controllers 206B are provided with eight transducers assigned to each slave controller 206B.

To electrically interconnect the control logic dies 206 and the transducers 212, in an embodiment, the flexible substrate 214 includes conductive traces 216 formed in the film layer that carry signals between the control logic dies 206 and the transducers 212. In particular, the conductive traces 216 providing communication between the control logic dies 206 and the transducers 212 extend along the flexible substrate 214 within the transition region 210. In some instances, the conductive traces 216 can also facilitate electrical communication between the master controller 206A and the slave controllers 206B. The conductive traces 216 can also provide a set of conductive pads that contact the conductors 218 of cable 142 when the conductors 218 of the cable 142 are mechanically and electrically coupled to the flexible substrate 214. Suitable materials for the conductive traces 216 include copper, gold, aluminum, silver, tantalum, nickel, and tin, and may be deposited on the flexible substrate 214 by processes such as sputtering, plating, and etching. In an embodiment, the flexible substrate 214 includes a chromium adhesion layer. The width and thickness of the conductive traces 216 are selected to provide proper conductivity and resilience when the flexible substrate 214 is rolled. In that regard, an exemplary range for the thickness of a conductive trace 216 and/or conductive pad is between 1-5 µm. For example, in an embodiment, 5 µm conductive traces 216 are separated by 5 µm of space. The width of a conductive trace 216 on the flexible substrate may be further determined by the width of the conductor 218 to be coupled to the trace/pad. The transmission line bundle or cable 112 can include a plurality of conductors, including one, two, three, four, five, six, seven, or more conductors 218.

The flexible substrate 214 can include a conductor interface 220 in some embodiments. The conductor interface 220 can be a location of the flexible substrate 214 where the conductors 218 of the cable 142 are coupled to the flexible substrate 214. For example, the bare conductors of the cable 142 are electrically coupled to the flexible substrate 214 at the conductor interface 220. The conductor interface 220 can be a tab extending from the main body of flexible substrate 214. In that regard, the main body of the flexible substrate 214 can refer collectively to the transducer region 204, controller region 208, and the transition region 210. In the illustrated embodiment, the conductor interface 220 extends from the proximal portion 222 of the flexible substrate 214. In other embodiments, the conductor interface 220 is positioned at other parts of the flexible substrate 214, such as the distal portion 221, or the flexible substrate 214 may lack the conductor interface 220. A value of a dimension of the tab or conductor interface 220, such as a width 224, can be less than the value of a dimension of the main body of the flexible substrate 214, such as a width 226. In some embodiments, the substrate forming the conductor interface 220 is made of the same material(s) and/or is similarly flexible as the flexible substrate 214. In other embodiments, the conductor interface 220 is made of different materials and/or is comparatively more rigid than the flexible substrate 214. For example, the conductor interface 220 can be made of a plastic, thermoplastic, polymer, hard polymer, etc., including polyoxymethylene (e.g., DELRIN^{®}), polyether ether ketone (PEEK), nylon, Liquid Crystal Polymer (LCP), and/or other suitable materials.

**Figure 3** illustrates a perspective view of the ultrasound scanner assembly 110 in a rolled configuration. In some instances, the assembly 110 is transitioned from a flat configuration (as shown for example in Figure 2) to a rolled or more cylindrical configuration (as shown for example in Figure 3). For example, in some embodiments, techniques are utilized as disclosed in one or more of U.S. Patent No. 6,776,763, titled "ULTRASONIC TRANSDUCER ARRAY AND METHOD OF MANUFACTURING THE SAME" and U.S. Patent No. 7,226,417, titled "HIGH RESOLUTION INTRAVASCULAR ULTRASOUND SENSING ASSEMBLY HAVING A FLEXIBLE SUBSTRATE".

In some embodiments, the transducer elements 212 and/or the controllers 206 can be positioned in in an annular configuration, such as a circular configuration or in a polygon configuration, around a longitudinal axis 250 of a support member 230. It will be understood that the longitudinal axis 250 of the support member 230 may also be referred to as the longitudinal axis of the scanner assembly 110, the flexible elongate member 121, and/or the intraluminal imaging device 102. For example, a cross-sectional profile of the imaging assembly 110 at the transducer elements 212 and/or the controllers 206 can be a circle or a polygon. Any suitable annular polygon shape can be implemented, such as a based on the number of controllers/transducers, flexibility of the controllers/transducers, etc., including a pentagon, hexagon, heptagon, octagon, nonagon, decagon, etc. In some examples, the plurality of transducer controllers 206 may be used for controlling the plurality of ultrasound transducer elements 212 to obtain imaging data associated with the vessel 120.

The support member 230 can be referenced as a unibody in some instances. The support member 230 can be composed of a metallic material, such as stainless steel, or non-metallic material, such as a plastic or polymer as described in U.S. Provisional Application No. 61/985,220, "Pre-Doped Solid Substrate for Intravascular Devices," filed April 28, 2014, ('220 Application, published as US 2015/0305710 A1)). The support member 230 can be a ferrule having a distal flange or portion 232 and a proximal flange or portion 234. The support member 230 can be tubular in shape and define a lumen 236 extending longitudinally therethrough. The lumen 236 can be sized and shaped to receive the guide wire 118. The support member 230 can be manufactured using any suitable process. For example, the support member 230 can be machined and/or electrochemically machined or laser milled, such as by removing material from a blank to shape the support member 230, or molded, such as by an injection molding process.

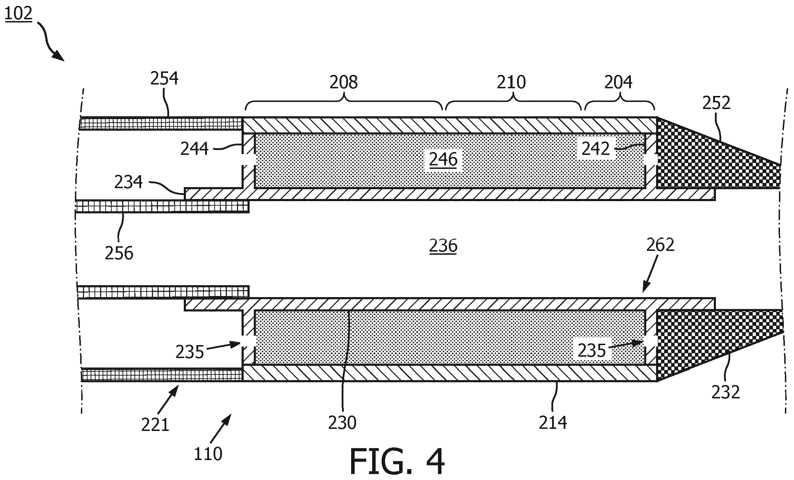

Figure 4 shows a diagrammatic cross-sectional side view of a distal portion of the intraluminal imaging device 102, including the flexible substrate 214 and the support member 230, according to aspects of the present disclosure. The support member 230 can be referenced as a unibody in some instances. The support member 230 can be composed of a metallic material, such as stainless steel, or non-metallic material, such as a plastic or polymer as described in U.S. Provisional Application No. 61/985,220, "Pre-Doped Solid Substrate for Intravascular Devices," filed April 28, 2014 (published as US 2015/0305710 A1). The support member 230 can be ferrule having a distal portion or flange 232 and a proximal or flange portion 234. The support member 230 can define a lumen 236 extending along the longitudinal axis LA. The lumen 236 is in communication with the entry/exit port 116 and is sized and shaped to receive the guide wire 118 (as shown for example in Figure 1). The support member 230 can be manufactured according to any suitable process. For example, the support member 230 can be machined and/or electrochemically machined or laser milled, such as by removing material from a blank to shape the support member 230, or molded, such as by an injection molding process. In some embodiments, the support member 230 may be integrally formed as a unitary or unibody structure, while in other embodiments the support member 230 may be formed of different components, such as a ferrule and stands 242, 244, that are fixedly coupled to one another. In some cases, the support member 230 and/or one or more components thereof may be completely integrated with an inner member or guide wire member 256. In some cases, the inner member 256 and the support member 230 may be joined as one, e.g., in the case of a polymer support member.

Stands 242, 244 that extend vertically are provided at the distal and proximal portions 232, 234, respectively, of the support member 230. The stands 242, 244 elevate and support the distal and proximal portions of the flexible substrate 214. In that regard, portions of the flexible substrate 214, such as the transducer portion 204 (or transducer region 204), can be spaced from a central body portion of the support member 230 extending between the stands 242, 244. The stands 242, 244 can have the same outer diameter or different outer diameters. For example, the distal stand 242 can have a larger or smaller outer diameter than the proximal stand 244 and can also have special features for rotational alignment as well as control chip placement and connection. To improve acoustic performance, any cavities between the flexible substrate 214 and the surface of the support member 230 are filled with a backing material 246. The liquid backing material 246 can be introduced between the flexible substrate 214 and the support member 230 via passageways 235 in the stands 242, 244. In some embodiments, suction can be applied via the passageways 235 of one of the stands 242, 244, while the liquid backing material 246 is fed between the flexible substrate 214 and the support member 230 via the passageways 235 of the other of the stands 242, 244. The backing material can be cured to allow it to solidify and set. In various embodiments, the support member 230 includes more than two stands 242, 244, only one of the stands 242, 244, or neither of the stands. In that regard the support member 230 can have an increased diameter distal portion 232 and/or increased diameter proximal portion 234 that is sized and shaped to elevate and support the distal and/or proximal portions of the flexible substrate 214.

The support member 230 can be substantially cylindrical in some embodiments. Other shapes of the support member 230 are also contemplated including geometrical, non-geometrical, symmetrical, non-symmetrical, cross-sectional profiles. As the term is used herein, the shape of the support member 230 may reference a cross-sectional profile of the support member 230. Different portions the support member 230 can be variously shaped in other embodiments. For example, the proximal portion 234 can have a larger outer diameter than the outer diameters of the distal portion 232 or a central portion extending between the distal and proximal portions 232, 234. In some embodiments, an inner diameter of the support member 230 (e.g., the diameter of the lumen 236) can correspondingly increase or decrease as the outer diameter changes. In other embodiments, the inner diameter of the support member 230 remains the same despite variations in the outer diameter.

An inner member 256 and a proximal outer member 254 are coupled to the proximal portion 234 of the support member 230. The inner member or guide wire member 256 and/or the proximal outer member 254 can comprise a flexible elongate member. The inner member 256 can be received within a proximal flange 234, or may terminate within the support member 230, or may extend entirely through the support member 230 and project out through the distal portion or flange 232. The proximal outer member 254 abuts and is in contact with the flexible substrate 214. A distal member 252 is coupled to the distal portion 232 of the support member 230. For example, the distal member 252 is positioned around the distal flange 232. The distal member 252 can abut and be in contact with the flexible substrate 214 and the stand 242. The distal member 252 can be the distal-most component of the intraluminal imaging device 102.

One or more adhesives can be disposed between various components at the distal portion of the intraluminal imaging device 102. For example, one or more of the flexible substrate 214, the support member 230, the distal member 252, the inner member 256, and/or the proximal outer member 254 can be coupled to one another via an adhesive.

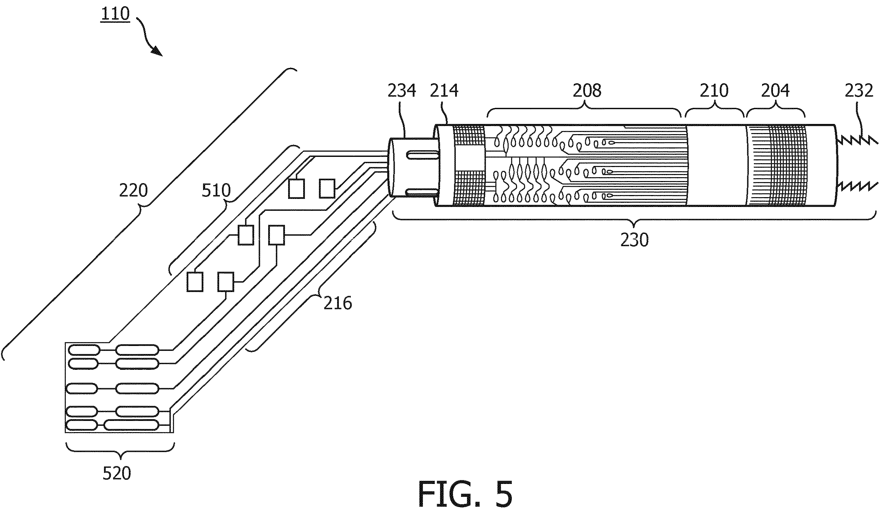

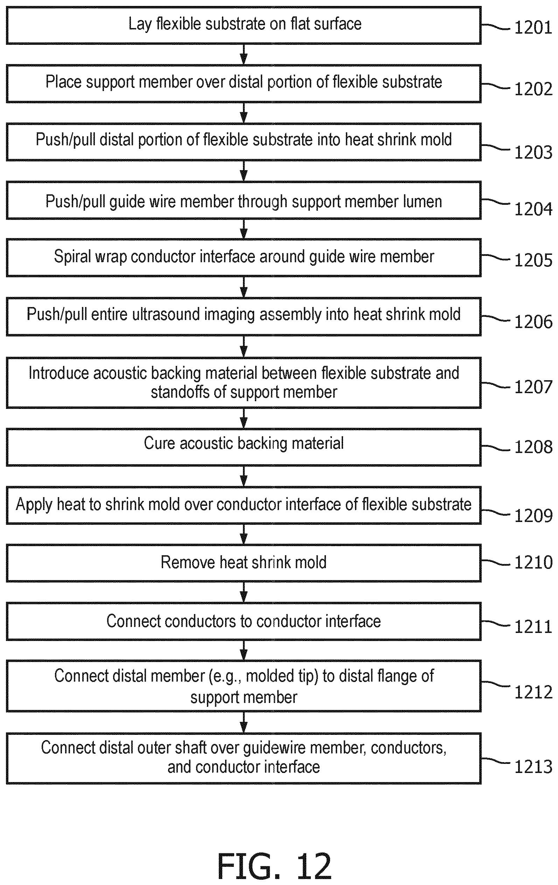

**Figure 5** is diagrammatic side view of an ultrasound imaging assembly 110 with a distal portion of a flexible substrate 214 in a rolled configuration around the support member 230, according to at least one embodiment of the present disclosure. The flexible scanner assembly 110 has been wrapped around the support member 230 (e.g., a ferrule, metal tube, unibody, or other suitable structure) such that the distal flange 232 and proximal flange 234 protrude, while the control region 208, transition region 210, and transducer region 204 have taken a cylindrical shape around the support member 230. In some instances, the control region 208, transition region 210, and transducer region 204 can be referenced as a distal portion of the flexible substrate 214. Also visible is the conductor interface 220, which includes conductive traces 216, electronic components 510, and conductive weld pads or solder pads 520 to which may be attached the conductors 218 shown in Figure 2. In some instances, the conductor interface 220 can be referenced as a proximal portion of the flexible substrate 214. The conductive traces 216 in the conductor interface 220 establish electrical communication between the weld pads 520 and the controller chips 206 in the controller region 208. In order to prevent heat damage to the scanner assembly 210 when conductors 218 are welded or soldered to the conductive pads 520 and when heat is applied to heat shrink tubing (see Figures 12, 16, and 18), the conductor interface 220 projects away from the control region 208 of the scanner assembly 110 for some distance at an exemplary angle of about 45 degrees, although other angles and distances may be used than those shown in this example.

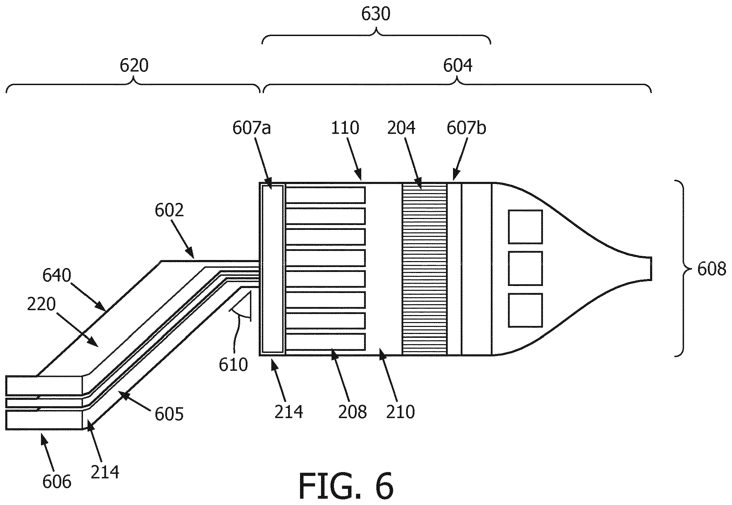

**Figure 6** is a top view of an example flexible scanner assembly 110 in its flat (unrolled) configuration, wherein the transducer region 204, control region 208, transition region 210, and conductor interface 220 have been fabricated on a flat, flexible substrate 214 in accordance with at least one embodiment of the present disclosure. In an example, the flex circuit 214 also includes two grounding regions 607a and 607b that correspond with the positions of the stands 242 and 244 of the support member 230, such that the stands 242 and 244 are not in mechanical or electrical contact with the electronic components of regions 204, 208, and 210 when the flexible scanner assembly 110 is in its rolled configuration as shown for example in Figures 3 and 5. In an embodiment, the grounding regions 607a and 607b serve as ground returns. For example, the regions 607a, 607b include conductive material that, e.g., when contacted to the stands 242, 244, serve as electric ground for the controllers 206 and/or the transducer elements 212. In some embodiments, the portions of the flexible substrate 214 distal of the grounding region 607b are removed after assembly such that they are not part of final assembled device.

In an example, the grounding regions 607a and 607b are separated by a distance 630, which may be between approximately 0.15 inches and approximately 0.2 inches, including values such as 0.185 inches (4.7 mm), and/or other suitable values both larger and smaller. The distance 630 may be length of the distal portion of the flexible substrate 214 in the final assembled device.

In some embodiments, the conductor interface 220 may be attached to the proximal grounding region 607a by an attachment leg 602, with a length of between approximately 0.04 inches and approximately 0.1 inches (1.0-2.5 mm). In other embodiments, the conductor interface 220 may project directly from the proximal grounding region 607a, or from the control region 208.

The distal portion of the flexible substrate 214 has a length 604 and a width 608. For example, the length 604 can be between approximately 0.18 inches and approximately 0.35 inches, including values such as 0.287 inches (7.3 mm), and/or other suitable values both larger and smaller. For example, the width 608 can be between approximately 0.1 inches and approximately 0.15 inches, including values such as 0.12 inches (3 mm), and/or other suitable values both larger and smaller. The length 604 and/or the width 608 can be based on the diameter of the intraluminal device. For example, the diameter can be between 2 Fr and 10 Fr in some embodiments, including values such as 3 Fr, 5 Fr, 8.5 Fr, and/or other suitable values both larger and smaller. The conductor interface 220 or proximal portion of the flexible substrate 214 has a length 620 and a width 640. For example, the length 620 can be between approximately 0.1 inches and approximately 0.5 inches (2.5-12.7 mm), including values such as 0.14 inches, 0.18 inches, 0.2 inches (.5 mm), and/or other suitable values both larger and smaller. For example, the width 640 can be between approximately 0.02 inches and approximately 0.05 inches, including values such as 0.022 inches (0.56 mm), 0.045 inches (1.14 mm), and/or other suitable values both larger and smaller. In that regard, the width 640 of the conductor interface 220 is less than the width 608 of the distal portion of the flexible substrate 214. In an example, each weld pad or solder pad at the proximal end of the conductor interface 220 has a width of approximately 0.00787 inches (0.2 mm). The proximal end of the conductor interface 220 can have a width different than central and/or distal portion of the conductor interface 220. For example, the width of the proximal end of the conductor interface 220 can be between approximately 0.04 inches and approximately 0.05 inches, including values such as approximately 0.043 inches (1.09 mm) and/or other suitable values both larger and smaller.

The conductor interface 220 projects at an oblique angle 610 from the distal portion of the flexible substrate. For example, the oblique angle 610 can be between approximately 1° and approximately 89°, between approximately 30° and approximately 75°, or between approximately 40° and approximately 50°, including values such as 30°, 40°, 45°, 50°, and 60°, and/or other suitable values both larger and smaller. The conductor interface 220 includes a longitudinal segment or attachment leg 602, an oblique segment 605, and a longitudinal segment 606.

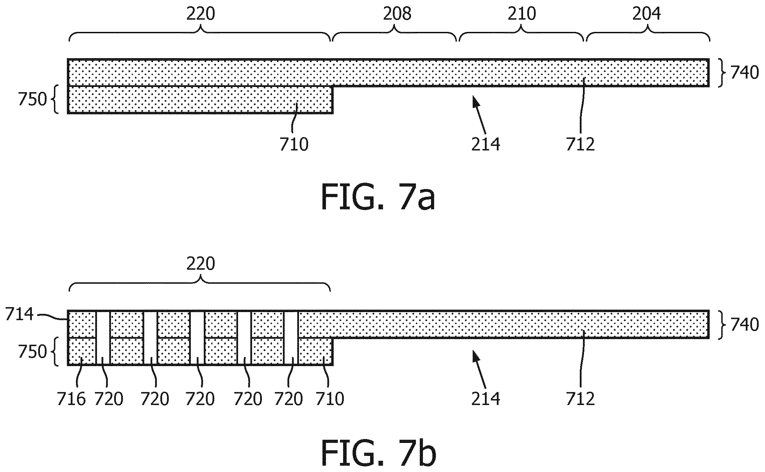

**Figure 7a** is a side diagrammatic view of the flexible substrate 214 of an ultrasound imaging assembly, according to at least one embodiment of the present disclosure. Figure 7a illustrates that the proximal portion of the flexible substrate 214 is formed to two layers 710, 712, while the distal portion of the flexible substrate 214 is formed of only one layer 712. In some embodiments, the conductor interface 220 includes an additional layer or cover layer 710 not found in the transducer region 204, control region 208, or transition region 210. In an example, both the layers 710, 712 are made the same material, such as polyimide (e.g., Kapton) or another polymer. The additional layer or cover layer 710 can advantageously serve to encapsulate the electrical traces within the conductor interface 220, to protect them from heat, handling, moisture, or fluid ingress. The electrical traces can extend between the layers 710, 712. In some embodiments, the layer 710 is formed of different material than the layer 712. In an example, the layer 712 has a thickness or height 740 between approximately 10 microns and approximately 15 microns, including values such as 12.5 microns, and/or other suitable values both larger and smaller. The layer 710 has a thickness or height 750 of between approximately 6 microns and approximately 25 microns, including values such as 15 microns, 20 microns, 25 microns. The greater total thickness or height of the approximately 18.5-37 microns makes the conductor interface 220 stiffer than the distal portion of the flex substrate 214. For example, the conductor interface 220 can be more difficult than the distal portion of the flexible substrate 214 to transition from the flat configuration into the rolled configuration because of the greater total thickness.

**Figure 7b** is a diagrammatic side view of the flexible substrate 214, including a plurality of recesses 720 in the conductor interface 220, according to at least one embodiment of the present disclosure. In the illustrated embodiment, the recesses 720 extend completely through the greater total thickness of the conductor interface 220 from a surface 714 to an opposite surface 716. The surface 714 forms part of the layer 712 and the surface 716 forms part of the layer 710. In an example, the recesses 720 are slits or through-holes that extend entirely through the layers 710, 712. The recesses 720 advantageously remove material from the thicker conductor interface 220, which aids in making the conductor interface 220 more flexible. For example, the flexibility of the conductor interface 220 with the recesses 720 can be similar to the flexibility of the distal portion of the flexible substrate 214. Because the conductor interface 220 is more flexible, transitioning the conductor interface 220 into the rolled configuration is easier. Efficiency of manufacturing of the ultrasound sensor assembly 110 is advantageously improved because the more flexible conductor interface 220 can be predictably rolled, e.g., in automated manner.

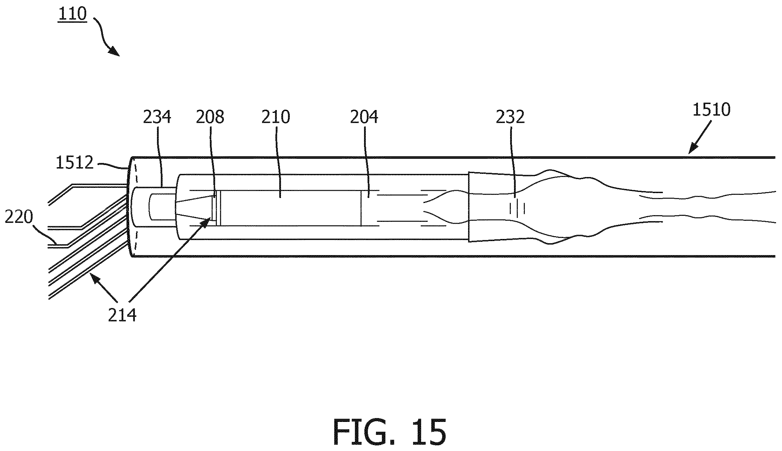

While the illustrated embodiment includes recesses 720, in general, the conductor interface 220 can generate a plurality of flexibility enhancements. The flexibility enhancements may be slits, voids, troughs, indentations, through-holes, or other localized removals of material, and may have one planar dimension larger than, smaller than, or substantially similar to a perpendicular planar dimension. The recesses or flexibility enhancements 720 can be incorporated in the flexible substrate 214 using any suitable cutting, dicing, etching method. The flexibility enhancements may be incorporated into either or both of the flexible substrate 214 and the extra layer 710, such that the flexibility of the conductor interface 220 is reduced, and may be comparable to the flexibility of the transducer region 204, control region 208, and transition region 210 as an aid to fabrication steps described below (see Figures 12 and 15). In some embodiments, the flexibility enhancements only extend partially through the thickness of the conductor interface 220.

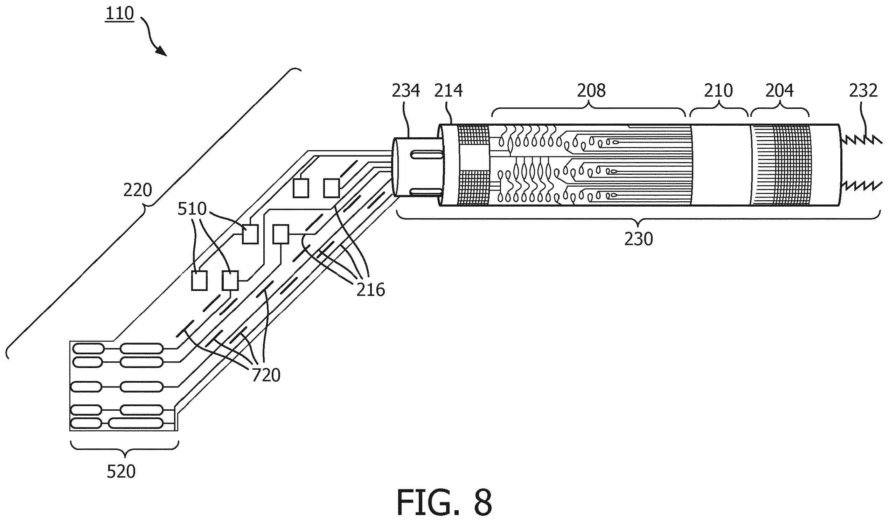

**Figure 8** is diagrammatic view of the ultrasound imaging assembly 110 with the distal portion of the flexible substrate 214 in a rolled configuration around the support member 230 and the proximal portion 220 including the recesses 720, according to at least one embodiment of the present disclosure. Dimensions and placement of the recesses 720 in the conductor interface 220 are selected such that the recesses 720 do not sever, expose, penetrate, short-circuit, render fragile, or otherwise disturb the conductive traces 216, electronic components 510, and conductive weld pads or solder pads 520.

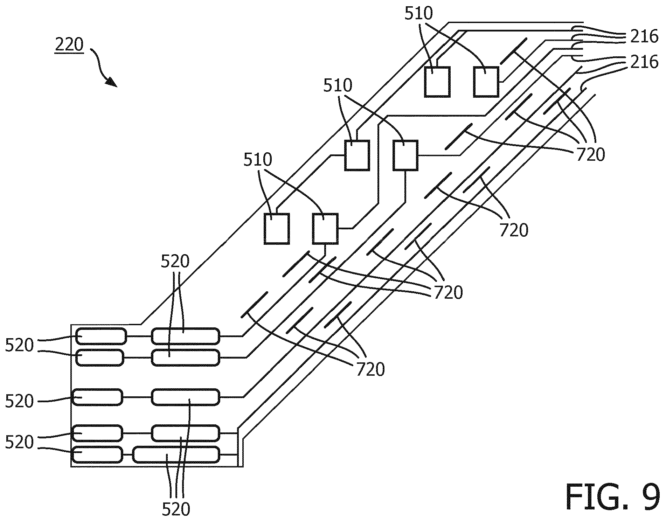

**Figure 9** is diagrammatic view of the proximal portion 220 of the flexible substrate 214. In this example, the recesses 720 are slits that are located in the interstitial spaces between the conductive traces 216, electronic components 510, and weld pads or solder pads 520. For example, the plurality of recesses 720 are spaced apart from one another in the proximal portion 220 of the flexible substrate 214 between the plurality of conductive traces 216. The slits 720 can have a length and extend parallel to the conductive traces 216. For example, a length of each slit 720 can be between approximately 0.005 inches and approximately 0.025 inches (0.127-0.635 mm), including values such as approximately 0.024 inches (0.6 mm). In an embodiment, the conductive traces 216 and the slits 720 extend in the same oblique angle as the conductor interface 220 extends from the distal portion of the flexible substrate 214. For example, the plurality of recesses 720 are arranged in a same orientation as the plurality of conductive traces 216. The one or more electronic components 510 in the conductor interface 220 can be any suitable active or passive electronic components. For example, the electronic components 510 may include capacitors and/or resistors that act on the ultrasound imaging signals from the transducer elements 212 (e.g., to filter or reduce noise in the ultrasound imaging signals). Each electrical component 510 is disposed along a path of a respective conductive trace 216 from the weld pads 520 to the controller chips 206.

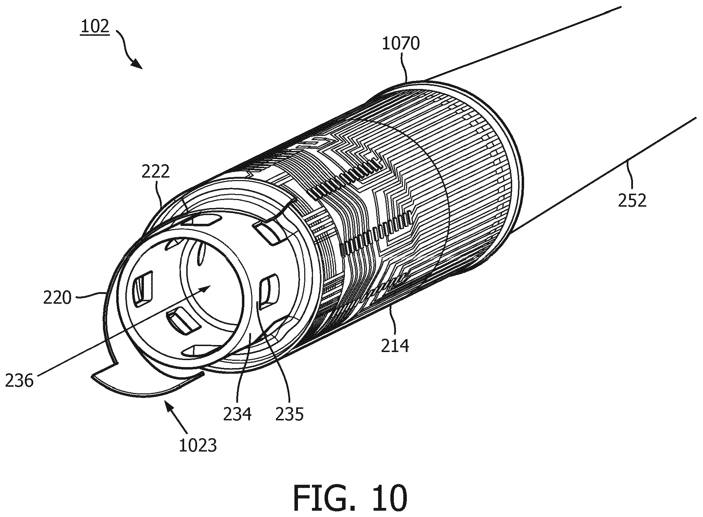

**Figure 10** is a diagrammatic perspective view of a distal portion of the intraluminal imaging device 102, including a distal portion of an imaging assembly, according to aspects of the present disclosure. The conductor interface 220 of the flexible substrate 214 can be positioned around the proximal flange 234. For example, the conductor interface 220 can be wrapped in a spiral or helical configuration around the proximal flange 234 such that the proximal portion 1023 of the conductor interface 220 is adjacent to the proximal flange 234. **In** other embodiments, the conductor interface 220 can extend proximally from the main body of the flex circuit in a different manner, such as a linear/straight configuration, a curved configuration, etc. Also visible are holes, apertures, or passageways 235 in the proximal flange 234 and support member 230, through which a backing material 246 (e.g., an epoxy in liquid or flowable form) may be introduced. Additionally visible is a distal member or tip 252, which may be attached to either or both of the distal flange 234 and the distal standoff 242 by means of an adhesive 1070.

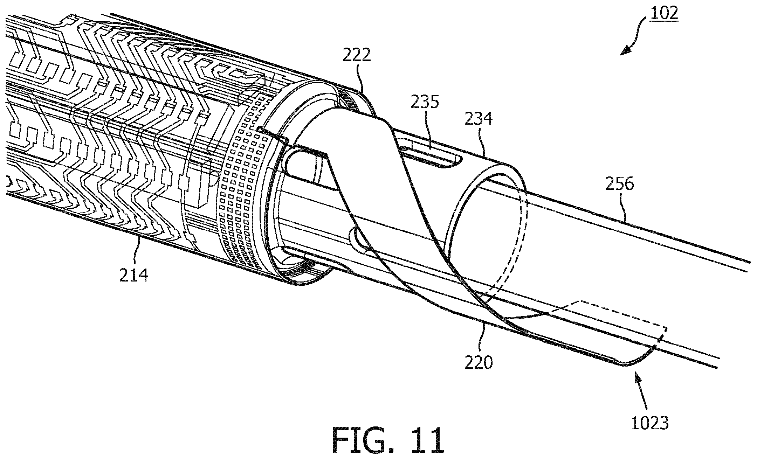

**Figure 11** is a diagrammatic perspective view of a distal portion of the intraluminal imaging device 102, including an ultrasound imaging assembly, according to aspects of the present disclosure. The conductor interface 220 of the flexible substrate 214 can be wound around the proximal flange 234 any suitable number of times, depending on the length of the conductor interface 220. In some embodiments, the proximal portion 1023 of the conductor interface 220 may extend to, and wrap around, an inner member or guide wire member 256. Also visible are holes or apertures 235 in the proximal flange 234.

**Figure 12** is a flow diagram of a method of assembling an intraluminal imaging device, according to aspects of the present disclosure. As illustrated, the method of Figure 12 includes a number of enumerated steps, but embodiments of the method may include additional steps before, after, and in between the enumerated steps. In some embodiments, one or more of the enumerated steps may be omitted, performed in a different order, or performed concurrently. The steps of the method can be carried out by a manufacturer of the intraluminal imaging device.

In step 1201, the flexible substrate 214 (which includes the transducer region 204, transition region 210, control region 208, and conductor interface 220) is laid on a flat work surface where additional steps may be performed.

In step 1202, the support member 230 is placed over the distal portion (e.g., regions 204, 208, and 210) of the flexible substrate 214. The support member 230 (e.g., a tube that may be a metallic ferrule and may have a unibody structure) may optionally be secured to the support member 230 with an adhesive.

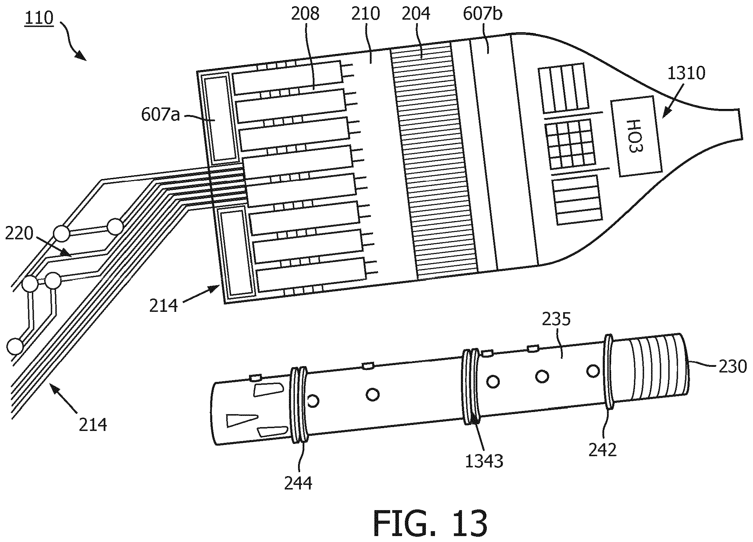

In step 1203, the distal portion of the flex circuit 214 (e.g., the regions 204, 208, 210) are moved into a plastic heat shrink mold to transition the flex circuit 214 from a flat configuration into a rolled configuration. Generally speaking, the heat shrink mold will be a cylindrical tube whose inner diameter exceeds the diameter of the support member 230 and its standoffs 242 and 244 by an amount greater than, e.g., twice the thickness of the layers 710, 712 of the flexible substrate 214, thus allowing the flexible substrate 214 to be rolled up around the support member 230 and fitted into the heat shrink mold. The insertion may be aided by a pointed or rounded shape 1310 (Figure 13) at the distal tip of the flexible substrate 214, adjacent to the transducer region 204. The pointed or rounded shape 1310 may include a narrow tip or header. The transducer region 204, the transition region 210 and control region 208 of the flex circuit 214 may be inserted into the mold by either pushing, pulling, or any combination thereof. When the flexible substrate 214, affixed to a cylindrical support member 230, is inserted into a cylindrical mold, engagement between one or more edges of the mold and one or more edges of the flexible substrate 214 will cause the flexible substrate to curl or roll around the support member 230 within the mold.

In step 1204, the inner member or guide wire member 256 is inserted through the lumen 236 of the support member 230. The guide wire member 256 allows the intraluminal imaging device 102 to be guided through a human blood vessel or other lumen 120 by a guide wire 118 (see Figure 1), and may also provide a form for the conductor interface to spiral around. In at least one alternative embodiment, the guide wire member 256 is not inserted at this time, but is rather inserted after heat is applied to the heat shrink mold in step 1209.

In step 1205, the conductor interface 220 or the proximal portion of the flexible substrate 214 is spirally wrapped or rolled. In some embodiments, the conductor interface 220 transitions to the spiral or rolled configuration within the heat shrink tubing. In some embodiments, the conductors interface 220 is spirally wrapped or rolled around the inner member or guide wire member 256 and/or the proximal flange 234 of the support member 230. In some embodiments, the step 1205 is performed immediately after the step 1203. For example, the conductor interface 220 can be inserted into the mold by either pushing, pulling, or any combination thereof, as a continuation of the transducer region 204, the transition region 210 and control region 208 being moved into the mold. Contact between an edge of the conductor interface 220 and the edge of the mold causes the conductor interface to enter the spiral or rolled configuration.

The recesses or flexibility enhancements 720 allow for the conductor interface 220 to be wrapped more efficiently. In an embodiment, the spiral wrapping is performed manually by a human operator or automatically by a machine. In some embodiments, the conductor interface 220 can be coupled to the inner member 256 by an adhesive. In that regard, because the conductor interface 220 has not yet been trained to retain its spiral configuration, it tries to return to its planar configuration. In that regard, the conductor interface 220 may be in a loose spiral configuration in the step 1207. The adhesive helps to maintain the spiral configuration before training is complete.

In step 1206, the ultrasound imaging assembly 110, including the conductor interface 220, is fully inserted into the heat shrink mold. In at least one embodiment, the conductor interface 220 has been wrapped, coiled, or otherwise positioned around the proximal flange 234 and/or the proximal side of the guide wire member 256 prior to this insertion. In at least one alternative embodiment, the inner member 256 has not yet been inserted, and the conductor interface 220 instead assumes a spiral configuration around the inner surface of the heat shrink mold as the ultrasound imaging assembly 110 is pushed or pulled into the mold. In this example, the recesses or flexibility enhancements 720 allow for the conductor interface 220 to curl and spiral more efficiently. In some embodiments, step 1205 is a part of step 1206 or vice versa.

In step 1207, the acoustic backing material 246 (e.g., in liquid or flowable form) is introduced into to the support member 230 in the region between one or more standoffs and the flexible substrate 214. The backing material 246 (shown in Figure 3) may serve to improve either or both of the mechanical stability and acoustic performance of the acoustic elements in the transducer region 204 (e.g., by limiting propagation of ultrasound energy in the undesired inward radial direction). The backing material 246 may optionally serve as the adhesive to couple to flexible substrate and the support member 230 as described in step 1202. In an example, the backing material 246 may be introduced through holes 235 (Figure 13) distributed along a length of the support member 230.

In step 1208, the heat shrink mold is placed in a curing environment such that the backing material 246 (e.g., a liquid compound) is cured into a substantially solid material as shown in Figure 4. In general curing may involve any combination of heat, light, UV, moisture, or chemical curing agents, although the heat of a curing oven is used in this example. Any heat energy or temperature change introduced to the backing material 246 during step 1208 is sufficient to cure the backing material 246 into a substantially solid material. In some embodiments, the curing does not cause heat shrink material to shrink so that there is no significant change in the size or other properties of the heat shrink mold.

In step 1209, heat energy is applied to the heat shrink mold using a heated die that applies heat to the region filled by the conductor interface 220, but not the control region 208, transition region 210, or transducer region 204 of the flexible substrate 214. The heat energy causes the heat shrink mold to shrink around the conductor interface 220, the proximal flange 234, and (if present) the inner member 256. The compression of the heat shrink mold and/or the heat trains the material of the conductor interface 220 to retain the shape it holds within the heat shrink mold. For example, if the conductor interface is spiraled around the proximal flange 234 and inner member or guide wire member 256, then the conductor interface will retain this shape when the heat shrink mold is removed. In other embodiments where the inner member 256 is not present during this step, the conductor interface 220 is spiraled around the inside of the heat shrink mold (e.g., the inside surface of a cylindrical tube), and as the heat is applied, the diameter of the heat shrink mold decreases, forcing the conductor interface 220 to form a tighter spiral. The combination of heat from the heated die and pressure from the heat shrink mold trains the material of the conductor interface 220 to retain this shape.

In step 1210, the heat shrink mold is removed, and may be discarded. The conductor interface 220 has now been trained to remember its shape.

In step 1211, the wires or conductors 218 are attached to the solder pads or weld pads 520 of the conductor interface 220. The attachment may be by methods including but not limited to soldering, welding, and conductive adhesive. The wires or conductors 218 may then be extended along the length of the inner member 256, wrapped around the inner member 256, and/or otherwise placed in a favorable configuration such that they do not interfere with the remaining assembly steps.

In step 1212, a distal member or tip 252 (e.g., a molded rubber or plastic tip) is attached to the distal flange of the support member. The distal member or tip 252 may be attached by any or all of snapping, screwing, welding, or adhesive.

In step 1213, a proximal outer member or shaft 254 is connected to the proximal flange 234 such that it fits over the inner member or guide wire member 256, conductors 218, and conductor interface 220. The proximal outer shaft may be connected by any or all of snapping, screwing, welding, or adhesive.

Figures 13-20 illustrate various steps in the method of Figure 12. **Figure 13** is a top view of the example flexible scanner assembly 110, with an example support member 230 alongside it, according to at least one embodiment of the present disclosure. In this example, the support member 230 is a tubular-shaped metallic unibody, although it could take other forms. In this example, the transducer region 204 of the flex circuit 214 includes a pointed tip 1310 that facilitates the insertion of the transducer region 204 into a heat shrink mold in accordance with step 1204. Also visible are the distal standoff 242 and proximal standoff 244, along with a central standoff 1343. Additionally visible are passageways 235 through which, in an example, a backing material 246 (e.g., a liquid compound) may be introduced. The ultrasound imaging assembly 110 is positioned on a flat surface in accordance with step 1201.

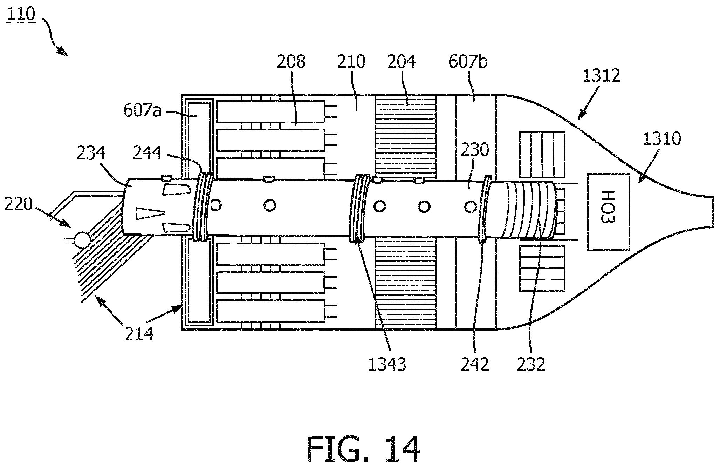

**Figure 14** is a top view of the example flexible scanner assembly 110 with the example tube unibody or support member 230 placed on top of the flexible substrate 214 in accordance with step 1202. The support member 230 overlies the transducer region 204, control region 208, and transition region 210 (the distal portion of the flexible substrate 214), but not the conductor interface 220 (the proximal portion of the flexible substrate 214). The unibody 230 may be secured in place by an adhesive. The standoff 244 is located proximally of the controller chips in the region 208, the stand 1343 is located in the transition region 210, and the standoff 242 is located distal of the acoustic elements in the transducer region 204.

**Figure 15** is a top view of a distal portion of a flexible substrate 214 of the ultrasound imaging assembly 110 inserted into the heat shrink mold 1510, in accordance with step 1203. For example, the tip 1310 can be pulled through the mold 1510. Engagement or contact of edges 1312 of the flexible substrate 214 with edges 1512 of the mold 1510 cause the flexible substrate 214 to transition from the flat configuration to the rolled configuration. In an example, the mold 1510 is a hollow cylinder, although non-circular cross sections such as squares, hexagons, and other polygons could be employed. In an example, the heat shrink mold 1510 is made from transparent or translucent plastic as a visual aid to the assembly process. In an example, the transducer region 204 has a pointed shape which aids in the insertion process, although other shapes may be used, including but not limited to rounded or squared. As the transducer region 204 is inserted into the cylindrical mold 1510, the flexible substrate curls up around the support member 230, such that as the support member 230 is further inserted into the mold, the transition region 210 and control region 208 also wrap around the support member 230, leaving the distal flange 232 and proximal flange 234 exposed within the mold 1510. The conductor interface 220 remains outside the heat shrink mold 1510.

At this point, the backing material 246 may be introduced and cured, and the heat shrink mold removed, in accordance with steps 1204 and 1205. This process transforms the distal portion of the flexible substrate 214 from the flat, configuration as seen in Figure 13 into a cylindrical or rolled configuration shown for example in Figure 5.

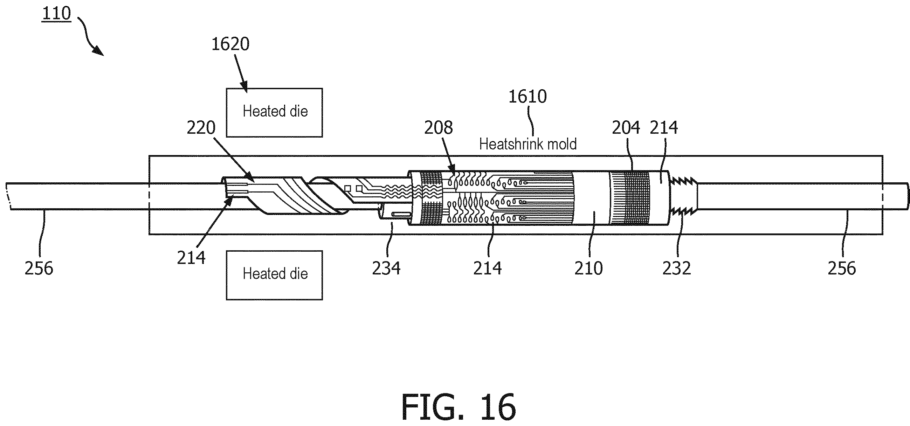

**Figure 16** is a side view of an example flexible scanner assembly 110 that has been fully inserted into a heat shrink mold 1610 in accordance with step 1208. In this example, flexibility enhancements 720 of the conductor interface 220 overcome the stiffness of the extra layer 710 of the conductor interface 220 as shown for example in Figure 7, such that in accordance with step 1208, the conductor interface 220 may be readily coiled, wrapped, or spiraled around an inner member or guide wire member 256 (step 1207) that has been inserted through the lumen 236 of the support member 230 (step 1206) and fitted into the heat shrink mold 1610. Also visible is a heated die 1620 in an open configuration.

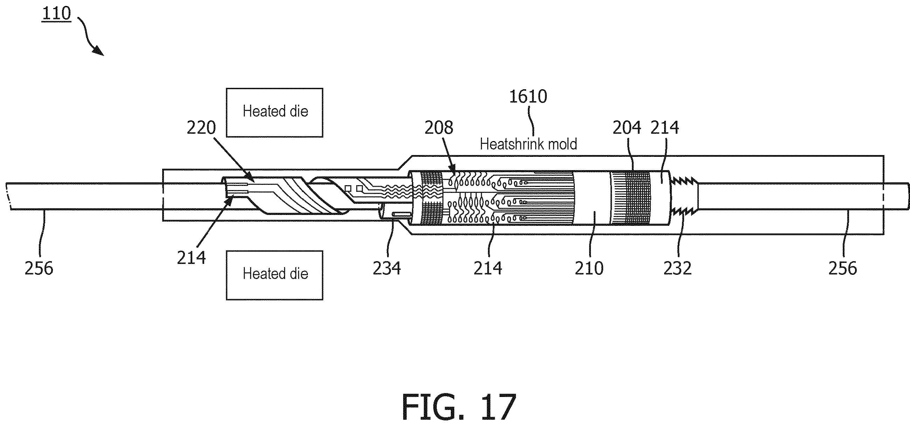

**Figure 17** is a side view of an example flexible scanner assembly, wherein in accordance with step 1209, the heated die 1620 has been closed around the proximal end of the heat shrink mold 1510, which encloses the conductor interface 220 wrapped around the inner member or guide wire member 256. Applied heat from the heated die 1620 causes the heat shrink mold 1610 to shrink, reducing both its outer diameter and inner diameter without substantially affecting its length. This creates a pressure on the conductor interface 220 which, combined with the applied heat from the heated die 1620, trains the material of the flexible substrate 214 of the conductor interface 220 to retain its shape, such that when the heat shrink mold 1610 is removed, the conductor interface 220 remains tightly coupled to the inner member or guide wire member 256. In an example, the conductor interface remains tightly coiled or spiraled around the inner member 256.

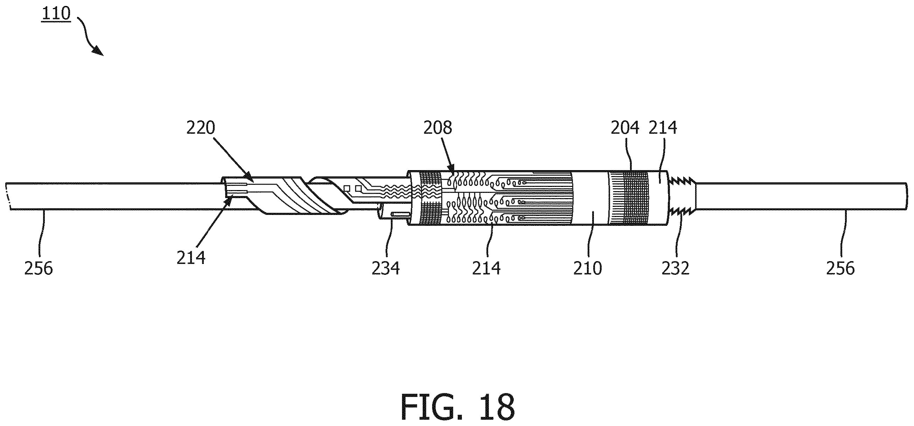

**Figure 18** is a side view of an example flexible scanner assembly 110 after the heat shrink mold 1610 has been removed, in accordance with step 1210. The flexible substrate material 214 of the conductor interface 220 has now been trained to retain substantially the shape it had while the heats shrink mold was compressed around it. In an example, the conductor interface 220 is coiled around the inner member or guide wire member 256, and will not spontaneously uncoil. The proximal portion 220 of the flexible substrate 214 has a spiral configuration around the inner member 256 and the distal portion of the flexible substrate 214 (regions 204, 208, 210) has a cylindrical configuration around the support member 230.

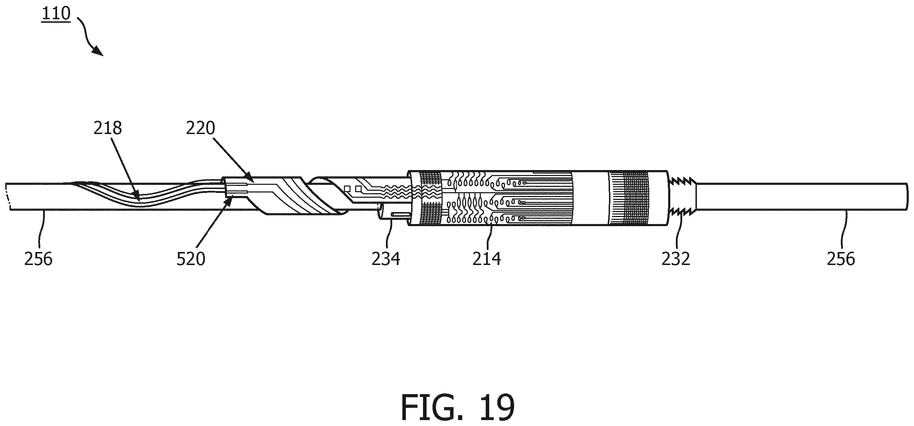

**Figure 19** is a side view of an example flexible scanner assembly 110 after the wires or conductors 218 of the cable or transmission line bundle 112 have been soldered or welded to the conductive pads 520 of the conductor interface 220, in accordance with step 1211. In that regard, the plurality of conductors 218 are coupled to the proximal portion 220 of the flexible substrate 214 at a location spaced between approximately 0.1 inches and approximately 0.5 inches (including values such as 0.2 inches or 5 mm, and other values both larger and smaller) from, e.g., the controller chips 206 at the distal portion of the flexible substrate 214. Accordingly, the welding or soldering is advantageously performed away from the controller chips so as to prevent any damage thereto, and a longer stiff length for the scanner assembly 110 is advantageously avoided. Moving the welding/soldering away from the interface between the imaging assembly and the outer member 254 also allows for a decreased diameter for the intravascular device. The increased flexibility of the conductor interface 220 afforded by the flexibility enhancements 720 also permits a decreased diameter for the scanner assembly 110, thus allowing the scanner assembly to be employed in narrower vessels. Electrically and/or mechanically coupling the conductors 218 to the conductor interface 220 establishes communication between the plurality of acoustic elements in the transducer region 204 and the plurality of electrical conductors 218 extending along a length of the flexible elongate member.

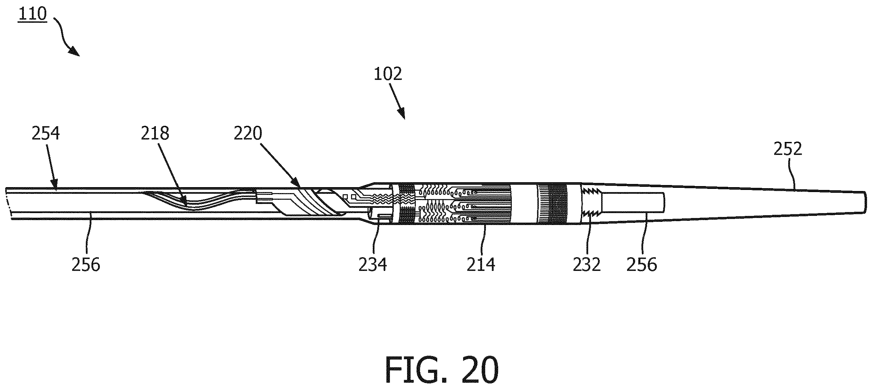

**Figure 20** is a side view of an example flexible scanner assembly 110 to which a proximal outer member 254 and distal member or distal tip have been added, in accordance with steps 1212 and 1213. The proximal outer member 254 may be attached to either or both of the proximal stand 244 or proximal flange 234 of the support member 230 by any combination of screwing, snapping, welding, or adhesive. The proximal outer member 254 encloses the inner member or guide wire member 256, the conductors 218 of the transmission line bundle or cable 112, the conductor interface 220, and the proximal portion or flange 234 of the support member 230. The distal member or distal tip 252 may be attached to either or both of the distal standoff 242 or distal flange 232 of the support member 230 by any combination of screwing, snapping, welding, or adhesive. The distal outer member 252 encloses the distal flange 232 of the support member 230. In an example, the distal member 252 is a molded rubber or plastic tip.

Persons skilled in the art, after becoming familiar with the teachings herein, will recognize that the apparatus, systems, and methods described above can be modified in various ways. Accordingly, persons of ordinary skill in the art will appreciate that the embodiments encompassed by the present disclosure are not limited to the particular exemplary embodiments described above. In that regard, although illustrative embodiments have been shown and described, a wide range of modification, change, and substitution is contemplated in the foregoing disclosure.

## Claims

1. An intraluminal imaging device (102), comprising:
a flexible elongate member (121) configured to be positioned within a body lumen of a patient, the flexible elongate member (121) comprising a proximal portion and a distal portion and a plurality of conductors extending along a length of the flexible elongate member;
an ultrasound imaging assembly disposed at the distal portion of the flexible elongate member (121), the ultrasound imaging assembly comprising:
a flexible substrate (214) comprising:
a distal portion (221) comprising a plurality of acoustic elements;
a proximal portion (222) configured to be coupled to the plurality of conductors (218) such that the plurality of conductors can communicate with the plurality of acoustic elements, the proximal portion comprising a plurality of flexibility enhancements in the form of localized removals of material, wherein the flexibility enhancements (720) extend partially through the thickness of the proximal portion or extend completely through the thickness of the flexible substrate (214) from a first surface to an opposite, second surface; and
a support member (230) around which the distal portion of the flexible substrate (214) is positioned.

2. The device of claim 1, wherein the proximal portion of the flexible substrate (214) comprises a first thickness greater than a second thickness of the distal portion of the flexible substrate (214).

3. The device of claim 2, wherein the distal portion of the flexible substrate (214) comprises a first layer, and the proximal portion of the flexible substrate (214) comprises the first layer and a second layer.

4. The device of claim 3, wherein the first layer comprises the first surface and the second layer comprises the second surface such that a plurality of recesses (720) extend completely through the first layer and the second layer.

5. The device of claim 3, wherein the first layer and the second layer comprise a same material.

6. The device of claim 1,
wherein the distal portion of the flexible substrate (214) comprises:
a plurality of integrated circuit chips in communication with the plurality of acoustic elements; and
a first plurality of conductive traces providing communication between the plurality of integrated circuit chips and the plurality of acoustic elements; and
wherein the proximal portion of the flexible substrate (214) comprises:
a plurality of conductive pads at which the plurality of conductors are coupled, respectively; and
a second plurality of conductive traces providing communication between the plurality of conductive pads and the plurality of integrated circuit chips.

7. The device of claim 6, wherein the plurality of recesses (720) are:
- spaced apart from one another in the proximal portion of the flexible substrate (214) between the second plurality of conductive traces; and/or
- arranged in a same orientation as the second plurality of conductive traces.

8. The device of claim 6, wherein the proximal portion of the flexible substrate (214) comprises one or more electrical components (510), wherein each of the one or more electrical components is disposed along a path (216) of a respective conductive trace of the second plurality of conductive traces.

9. The device of claim 1, wherein the proximal portion of the flexible substrate (214) comprises a first width less than a second width of the distal portion of the flexible substrate.

10. The device of claim 1, wherein the distal portion of the flexible substrate (214) comprises a cylindrical configuration around the support member (230), and wherein the proximal portion of the flexible substrate (214) comprises a spiral configuration.

11. The device of claim 10, wherein the flexible elongate member (121) comprises an inner member (256), wherein the proximal portion of the flexible substrate (214) comprises the spiral configuration around the inner member (256).

12. The device of claim 10, wherein the spiral configuration is trained into the proximal portion of the flexible substrate (214) by either or both of heat or compression.

13. The device of claim 1, wherein the proximal portion of the flexible substrate (214) extends at an oblique angle relative to the distal portion of the flexible substrate (214).

14. A method of assembling an intraluminal imaging device, the method comprising:
providing an ultrasound imaging assembly comprising a flexible substrate in a flat configuration, the flexible substrate comprising:
a distal portion comprising a plurality of acoustic elements; and
a proximal portion configured to be coupled to a plurality of conductors such that the plurality of conductors can communicate with the plurality of acoustic elements, the proximal portion comprising a plurality of flexibility enhancements in the form of localized removals of material, wherein the flexibility enhancements extend partially through the thickness of the proximal portion or extend completely through the thickness of flexible substrate from a first surface of the flexible substrate to an opposite, second surface of the flexible substrate;
transitioning the flexible substrate from the flat configuration into a rolled configuration, wherein the plurality of flexibility enhancements increase a flexibility of the proximal portion for the proximal portion to transition into the rolled configuration;
coupling the ultrasound imaging assembly to a distal portion of a flexible elongate member configured to be inserted into a body lumen of a patient; and
establishing communication between the plurality of acoustic elements and a plurality of electrical conductors extending along a length of the flexible elongate member, wherein establishing communication comprises coupling the plurality of electrical conductors to the proximal portion of the flexible substrate.

15. The method of claim 14, wherein transitioning comprises:
rolling the distal portion of the flexible substrate into a cylindrical configuration; and
rolling the proximal portion of the flexible substrate into a spiral configuration; and
wherein, preferably, transitioning comprises:
training the proximal portion of flexible substrate to retain the spiral configuration; and
wherein, further preferably, training comprises:
inserting the proximal portion of the flexible substrate into a heat shrink mold; and
applying heat such that the heat shrink mold compresses the proximal portion of the flexible substrate in the spiral configuration.

## Patentansprüche

1. Intraluminale Bildgebungsvorrichtung (102), umfassend:
ein flexibles, längliches Glied (121), welches so konfiguriert ist, dass es im Körperlumen eines Patienten positioniert werden kann, wobei das flexible, längliche Glied (121) einen proximalen Abschnitt und einen distalen Abschnitt und eine Vielzahl von Leitern umfasst, welche sich entlang einer Länge des flexiblen, länglichen Glieds erstrecken;
eine Ultraschallbildgebungsanordnung, welche an dem distalen Abschnitt des flexiblen, länglichen Glieds (121) angeordnet ist, wobei die Ultraschallbildgebungsanordnung umfasst:
ein flexibles Substrat (214), umfassend:
einen distalen Abschnitt (221), welcher eine Vielzahl von akustischen Elementen umfasst;
einen proximalen Abschnitt (222), welcher so konfiguriert ist, dass er mit der Vielzahl von Leitern (218) gekoppelt werden kann, sodass die Vielzahl von Leitern mit der Vielzahl von akustischen Elementen kommunizieren kann, wobei der proximale Abschnitt eine Vielzahl von Flexibilitätsverbesserungen in Form von lokalisierten Materialabtragungen umfasst, wobei sich die Flexibilitätsverbesserungen (720) teilweise durch die Dicke des proximalen Abschnitts erstrecken oder komplett durch die Dicke des flexiblen Substrats (214) von einer ersten Oberfläche zu einer gegenüberliegenden zweiten Oberfläche erstrecken; und
ein Stützglied (230), rund um welches der distale Abschnitt des flexiblen Substrats (214) positioniert ist.

2. Vorrichtung nach Anspruch 1, wobei der proximale Abschnitt des flexiblen Substrats (214) eine erste Dicke umfasst, welche größer ist als eine zweite Dicke des distalen Abschnitts des flexiblen Substrats (214).

3. Vorrichtung nach Anspruch 2, wobei der distale Abschnitt des flexiblen Substrats (214) eine erste Schicht umfasst, und der proximale Abschnitt des flexiblen Substrats (214) die erste Schicht und eine zweite Schicht umfasst.

4. Vorrichtung nach Anspruch 3, wobei die erste Schicht die erste Oberfläche umfasst und die zweite Schicht die zweite Oberfläche umfasst, sodass sich eine Vielzahl von Aussparungen (720) komplett durch die erste Schicht und die zweite Schicht erstrecken.

5. Vorrichtung nach Anspruch 3, wobei die erste Schicht und die zweite Schicht dasselbe Material umfassen.

6. Vorrichtung nach Anspruch 1,
wobei der distale Abschnitt des flexiblen Substrats (214) umfasst:
eine Vielzahl von IC-Chips in Kommunikation mit der Vielzahl von akustischen Elementen; und
eine erste Vielzahl von Leiterbahnen, welche Kommunikation zwischen der Vielzahl von IC-Chips und der Vielzahl von akustischen Elementen bereitstellen; und
wobei der proximale Abschnitt des flexiblen Substrats (214) umfasst:
eine Vielzahl von leitenden Pads, an welche jeweils die Vielzahl von Leitern gekoppelt sind; und
eine zweite Vielzahl von Leiterbahnen, welche Kommunikation zwischen der Vielzahl von leitenden Pads und der Vielzahl von IC-Chips bereitstellen.

7. Vorrichtung nach Anspruch 6, wobei die Vielzahl der Aussparungen (720) sind:
- in dem proximalen Abschnitt des flexiblen Substrats (214) zwischen der zweiten Vielzahl von Leiterbahnen voneinander beabstandet; und/oder
- in derselben Ausrichtung wie die zweite Vielzahl von Leiterbahnen angeordnet.

8. Vorrichtung nach Anspruch 6, wobei der proximale Abschnitt des flexiblen Substrats (214) eine oder mehrere elektrische Komponenten (510) umfasst, wobei jede der einen oder mehreren elektrischen Komponenten entlang eines Pfades (216) einer jeweiligen Leiterbahn der zweiten Vielzahl von Leiterbahnen angeordnet ist.

9. Vorrichtung nach Anspruch 1, wobei der proximale Abschnitt des flexiblen Substrats (214) eine erste Breite umfasst, welche kleiner ist als eine zweite Breite des distalen Abschnitts des flexiblen Substrats.

10. Vorrichtung nach Anspruch 1, wobei der distale Abschnitt des flexiblen Substrats (214) eine zylindrische Konfiguration rund um das Stützglied (230) umfasst, und wobei der proximale Abschnitt des flexiblen Substrats (214) eine spiralförmige Konfiguration umfasst.

11. Vorrichtung nach Anspruch 10, wobei das flexible längliche Glied (121) ein inneres Glied (256) umfasst, wobei der proximale Abschnitt des flexiblen Substrats (214) die spiralförmige Konfiguration rund um das innere Glied (256) umfasst.

12. Vorrichtung nach Anspruch 10, wobei die spiralförmige Konfiguration durch eines oder beides von Wärme oder Kompression in den proximalen Abschnitt des flexiblen Substrats (214) eingeformt wird.

13. Vorrichtung nach Anspruch 1, wobei sich der proximale Abschnitt des flexiblen Substrats (214) in einem schrägen Winkel relativ zu dem distalen Abschnitt des flexiblen Substrats (214) erstreckt.

14. Verfahren zum Montieren einer intraluminalen Bildgebungsvorrichtung, wobei das Verfahren umfasst:
Bereitstellen einer Ultraschallbildgebungsanordnung, welche ein flexibles Substrat in einer flachen Konfiguration umfasst, wobei das flexible Substrat umfasst:
einen distalen Abschnitt, welcher eine Vielzahl von akustischen Elementen umfasst; und
einen proximalen Abschnitt, welcher so konfiguriert ist, dass er mit einer Vielzahl von Leitern gekoppelt werden kann, sodass die Vielzahl von Leitern mit der Vielzahl von akustischen Elementen kommunizieren kann, wobei der proximale Abschnitt eine Vielzahl von Flexibilitätsverbesserungen in Form von lokalisierten Materialabtragungen umfasst, wobei sich die Flexibilitätsverbesserungen teilweise durch die Dicke des proximalen Abschnitts erstrecken oder komplett durch die Dicke von flexiblem Substrat von einer ersten Oberfläche des flexiblen Substrats zu einer gegenüberliegenden zweiten Oberfläche des flexiblen Substrats erstrecken;
Überführen des flexiblen Substrats von der flachen Konfiguration in eine gerollte Konfiguration, wobei die Vielzahl von Flexibilitätsverbesserungen eine Flexibilität des proximalen Abschnitts erhöht, um der proximale Abschnitt in die gerollte Konfiguration zu überführen;
Koppeln der Ultraschallbildgebungsanordnung an einen distalen Abschnitt eines flexiblen, länglichen Glieds, welches so konfiguriert ist, dass es in ein Körperlumen eines Patienten eingeführt werden kann; und
Herstellen einer Kommunikation zwischen der Vielzahl von akustischen Elementen und einer Vielzahl von elektrischen Leitern, welche sich entlang einer Länge des flexiblen, länglichen Glieds erstrecken, wobei Herstellen einer Kommunikation Koppeln der Vielzahl von elektrischen Leitern an den proximalen Abschnitt des flexiblen Substrats umfasst.

15. Verfahren nach Anspruch 14, wobei Überführen umfasst:
Rollen des distalen Abschnitts des flexiblen Substrats in eine zylindrische Konfiguration; und
Rollen des proximalen Abschnitts des flexiblen Substrats in eine spiralförmige Konfiguration; und
wobei Überführen bevorzugt umfasst:
Einformen des proximalen Abschnitts des flexiblen Substrats, um die spiralförmige Konfiguration beizubehalten; und
wobei Einformen weiter bevorzugt umfasst:
Einführen des proximalen Abschnitts des flexiblen Substrats in eine wärme-schrumpfende Gussform; und
Anwenden von Wärme, sodass die wärme-schrumpfende Gussform den proximalen Abschnitt des flexiblen Substrats in die spiralförmige Konfiguration komprimiert.

## Revendications

1. Dispositif d'imagerie intraluminale (102), comprenant :
un élément allongé flexible (121) configuré pour être positionné dans la lumière corporelle d'un patient, l'élément allongé flexible (121) comprenant une partie proximale et une partie distale et une pluralité de conducteurs s'étendant sur toute la longueur de l'élément allongé flexible ;
un ensemble d'imagerie par ultrasons disposé à l'extrémité distale de l'élément allongé flexible (121), cet ensemble d'imagerie par ultrasons comprenant :
un substrat flexible (214) comprenant :
une partie distale (221) comprenant une pluralité d'éléments acoustiques ;
une partie proximale (222) configurée pour être couplée à la pluralité de conducteurs (218) de sorte que la pluralité de conducteurs puisse communiquer avec la pluralité d'éléments acoustiques, la partie proximale comprenant une pluralité d'améliorations de flexibilité sous forme d'enlèvements localisés de matière, dans lequel les améliorations de flexibilité (720) s'étendent partiellement à travers l'épaisseur de la partie proximale ou s'étendent complètement à travers l'épaisseur du substrat flexible (214) d'une première surface à une seconde surface opposée ; et
un élément de support (230) autour duquel est positionnée la partie distale du substrat flexible (214).

2. Dispositif selon la revendication 1, dans lequel la partie proximale du substrat flexible (214) comprend une première épaisseur supérieure à une seconde épaisseur de la partie distale du substrat flexible (214).

3. Dispositif selon la revendication 2, dans lequel la partie distale du substrat flexible (214) comprend une première couche, et la partie proximale du substrat flexible (214) comprend la première couche et une seconde couche.

4. Dispositif selon la revendication 3, dans lequel la première couche comprend la première surface et la seconde couche comprend la seconde surface de sorte qu'une pluralité de cavités (720) s'étendent complètement à travers la première couche et la seconde couche.

5. Dispositif selon la revendication 3, dans lequel la première couche et la seconde couche comprennent un même matériau.

6. Dispositif selon la revendication 1,
dans lequel la partie distale du substrat flexible (214) comprend :
une pluralité de puces de circuits intégrés en communication avec la pluralité d'éléments acoustiques ; et
une première pluralité de pistes conductrices assurant la communication entre la pluralité de puces de circuits intégrés et la pluralité d'éléments acoustiques ; et
dans lequel la partie proximale du substrat flexible (214) comprend :
une pluralité de plots conducteurs auxquels sont respectivement connectés les différents conducteurs ; et
une seconde pluralité de pistes conductrices assurant la communication entre la pluralité de plots conducteurs et la pluralité de puces de circuits intégrés.

7. Dispositif selon la revendication 6, dans lequel la pluralité d'évidements (720) sont :
- espacés les uns des autres dans la partie proximale du substrat flexible (214) entre la seconde pluralité de pistes conductrices ; et/ou
- disposés dans une même orientation que la seconde pluralité de pistes conductrices.

8. Dispositif selon la revendication 6, dans lequel la partie proximale du substrat flexible (214) comprend un ou plusieurs composants électriques (510), dans lequel chacun des composants électriques est disposé le long d'un chemin (216) d'une piste conductrice respective de la seconde pluralité de pistes conductrices.

9. Dispositif selon la revendication 1, dans lequel la partie proximale du substrat flexible (214) comprend une première largeur inférieure à une seconde largeur de la partie distale du substrat flexible.

10. Dispositif selon la revendication 1, dans lequel la partie distale du substrat flexible (214) comprend une configuration cylindrique autour de l'élément de support (230), et dans lequel la partie proximale du substrat flexible (214) comprend une configuration en spirale.

11. Dispositif selon la revendication 10, dans lequel l'élément allongé flexible (121) comprend un élément intérieur (256), dans lequel la partie proximale du substrat flexible (214) comprend la configuration en spirale autour de l'élément intérieur (256).

12. Dispositif selon la revendication 10, dans lequel la configuration en spirale est entraînée dans la partie proximale du substrat flexible (214) par la chaleur ou la compression, ou les deux.

13. Dispositif selon la revendication 1, dans lequel la partie proximale du substrat flexible (214) s'étend selon un angle oblique par rapport à la partie distale du substrat flexible (214).

14. Procédé d'assemblage d'un dispositif d'imagerie intraluminale, le procédé comprenant :
la fourniture d'un ensemble d'imagerie par ultrasons comprenant un substrat flexible en configuration plate, le substrat flexible comprenant :
une partie distale comprenant une pluralité d'éléments acoustiques ; et
une partie proximale configurée pour être couplée à une pluralité de conducteurs de sorte que la pluralité de conducteurs puisse communiquer avec la pluralité d'éléments acoustiques, la partie proximale comprenant une pluralité d'améliorations de flexibilité sous la forme d'enlèvements localisés de matière, dans lequel les améliorations de flexibilité s'étendent partiellement à travers l'épaisseur de la partie proximale ou s'étendent complètement à travers l'épaisseur du substrat flexible d'une première surface du substrat flexible à une seconde surface opposée du substrat flexible ;
la transition du substrat flexible de la configuration plate à une configuration enroulée, dans lequel la pluralité d'améliorations de flexibilité augmente la flexibilité de la partie proximale pour que celle-ci puisse passer à la configuration enroulée ;
le raccordement du système d'imagerie par ultrasons à une partie distale d'un élément flexible et allongé conçu pour être inséré dans la lumière corporelle d'un patient ; et
l'établissement d'une communication entre la pluralité d'éléments acoustiques et une pluralité de conducteurs électriques s'étendant sur toute la longueur de l'élément allongé flexible, dans lequel l'établissement de la communication comprend le couplage de la pluralité de conducteurs électriques à la partie proximale du substrat flexible.

15. Procédé selon la revendication 14, dans lequel la transition comprend :
l'enroulement de la partie distale du substrat flexible en une configuration cylindrique ; et
l'enroulement de la partie proximale du substrat flexible en une configuration en spirale ; et
dans lequel, de préférence, la transition comprend :
l'entraînement de la partie proximale du substrat flexible à conserver la configuration en spirale ; et
dans lequel, de préférence, l'entraînement comprend :
l'insertion de la partie proximale du substrat flexible dans un moule thermorétractable ; et
l'application de la chaleur de sorte que le moule thermorétractable comprime la partie proximale du substrat flexible en configuration en spirale.
